(19) Europäisches Patentamt
European Patent Office
Office européen des brevets

(11) Publication number : **0 467 699 A2**

# (12) EUROPEAN PATENT APPLICATION

(21) Application number : **91306597.5**

(22) Date of filing : **19.07.91**

(51) Int. Cl.[5] : **C07K 7/06,** C07K 7/08, C12P 21/04, A61K 37/02, A61K 39/21

(30) Priority : **19.07.90 US 555227**

(43) Date of publication of application : **22.01.92 Bulletin 92/04**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Hannah, John**
**155 Idlebrook Lane**
**Matawan, NJ 07747 (US)**
Inventor : **Tolman, Richard L.**
**29 Upper Warren Way**
**Warren, NJ 07059 (US)**

(74) Representative : **Barrett-Major, Julie Diane et al**
**Merck & Co., Inc. European Patent Department Terlings Park Eastwick Road**
**Harlow Essex CM20 2QR (GB)**

## (54) Cyclic HIV principal neutralizing determinant peptides.

(57) Human Immunodeficiency Virus (HIV) Principal Neutralizing Determinant (PND) peptides, or peptides immunologically equivalent therewith, having the structure :

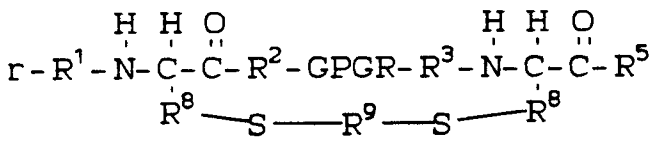

wherein the ring system contains stable thioether bonds, are useful as analytical tools, as reagents in ELISA assays, or as reagents for making covalent conjugate immunogens. Conjugates containing these stable, cyclic peptides are useful for raising mammalian anti-peptide, anti-HIV, or HIV-neutralizing immune responses, and a composition containing such a conjugate may be used as a vaccine to prevent HIV disease, including Acquired Immune Deficiency Syndrome, (AIDS), or AIDS related complex, (ARC), or as an immunogen for treating humans afflicted with AIDS or ARC.

EP 0 467 699 A2

## BACKGROUND OF THE INVENTION

Human Immunodeficiency Virus (HIV) principal neutralizing determinant (PND) peptides have been described which are capable of raising an anti-HIV immune response in mammals. The hypervariable region of the AIDS virus envelope glycoprotein, gp120, between amino acids 296 and 341 [according to the numbering scheme of Ratner et al., Nature 313, 277 (1985)] contains the amino acid tetramer -GlyProGlyArg- (-GPGR-) in most of the HIV isolates identified to date. In addition, a cysteine residue is generally found within about 20 amino acids on either side of this tetramer. In at least one common isolate, HIV IIIB, it is known that these cysteines are disulfide bonded. Thus, the intermediate amino acids, herein referred to as loop amino acids, are forced into a cyclic structure, with the -GPGR- being exposed at the loop-tip [Javaherian et al., PNAS USA 86, 6768, (1989)].

Peptide based efforts aimed at the induction of HIV neutralizing immune responses in mammals have generally been limited to the use of linear or disulfide bonded cyclic peptides. The linear peptides have the limitation that the recipient immune system is exposed to an epitope which constantly changes its three dimensional conformation in solution, while reliance on disulfide bonding to limit the number of conformations assumable by the linear PNDs is not completely satisfactory as disulfides are labile. Breakage of the disulfides results in the linear peptide having the attendant problems described above.

In addition to the problems associated with linear or disulfide bonded peptides noted above, an additional problem is that a great many different isolates of the HIV have been identified wherein sequence divergence of the PND has been noted. This diversity implies that if PNDs producing isolate specific immune responses are to be effective anti-HIV immunogens, then a mixture of PND peptides from different isolates may be the only solution to attainment of a broadly therapeutic or protective immunogen. In order to better characterize effective PND secondary structures, stable cyclics which avoid the use of labile disulfide bonding are needed. The cyclic peptides and the process for making these products disclosed in the instant invention satisfy this need.

Linear synthetic peptides have been prepared by a number of strategies conducted either in solution or on solid supports. Excellent texts covering the principles and techniques involved are listed: Principles of Peptide Synthesis, Bodanszky. M., Springer-Verlag (1984); Solid Phase Peptide Synthesis, Stewart J. M., Young, J. D., Pierce Chemical Company (2nd. ed. 1984); The Peptides, Gross, E., Meienhofer, J., Academic Press, Inc., (1979). In general, where cyclic peptides are required, disulfide-bonded cyclics have been prepared by oxidation of linear synthetic peptides containing at least two sulfhydryls, for example, peptides containing two cysteines. The instant invention overcomes the problems associated with the use of labile disulfides by providing peptides having stable, thioether-bonded cyclic structures.

Thus, this invention discloses novel HIV PND peptides having stable cyclic structures, a process for making, and a method of using such compounds. The stable cyclic HIV PND peptides, cPNDs, are prepared by formation of a thioether bonded bridge through cysteines on ether side of the loop amino acids. The cPNDs so formed are useful reagents for preparing covalent conjugate immunogens containing the cPNDs. Such conjugates are useful for raising mammalian anti-peptide, anti-HIV, or HIV-neutralizing immune responses. Compositions containing such conjugates may be used as a vaccine to prevent HIV disease, including Acquired Immune Deficiency Syndrome, (AIDS), or AIDS related complex (ARC) or as an immunogen for treating humans afflicted with AIDS or ARC. In addition, such conjugates provide a useful laboratory tool for analyzing the structure-function relationship involved in peptide elicitation of mammalian HIV-neutralizing immune responses.

## SUMMARY OF THE INVENTION

This invention is concerned with novel, stable, cyclic HIV PND peptides having the structure:

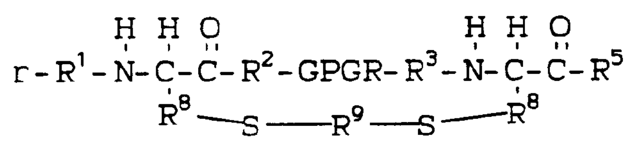

or pharmaceutically acceptable salts thereof, wherein:

r is:

a) hydrogen,
b) benzyloxycarbonyl,
c) Ac-Cys, or

d) Ac-Cys(Acm);
$R^1$ is:
a) a bond, or
b) a peptide of 1 to 5 amino acids, optionallly including a marker amino acid;
$R^2$ is :
a) either a bond or a peptide of up to 17 amino acids if $R^3$ is a peptide of at least 2 amino acids, or
b) a peptide of between 2 to 17 amino acids, if $R^3$ is a bond;
$R^3$ is:
a) either a bond or a peptide of up to 17 amino acids if $R^2$ is a peptide of at least 2 amino acids, or
b) a peptide of between 2 to 17 amino acids, if $R^2$ is a bond;
-GPGR- is the tetramer -GlyProGlyArg-;
$R^5$ is:
a) -OH,
b) a peptide of 1 to 5 amino acids, optionally including a marker amino acid,
c) $-NH_2$;
$R^8$ is lower alkyl of between one and eight carbons;
$R^9$ is:
a) $R^{10}$, or
b) xylylene; and
$R^{10}$ is:
a) lower alkyl of between one and eight carbons, or
b) $-CH_2-O-CH_2-$.

Peptides of this invention are prepared by cyclizing linear peptides made by solid phase chemical synthesis incorporating protective group chemistry. Cyclization is achieved by inclusion, during synthesis of the linear peptide, of reactive sites having labile protecting groups on the amino- and carboxy-terminal sides of the loop amino acids. Thus, in one embodiment of the invention, the sulfurs of cysteines incorporated into the peptide on either side of the loop amino acids are reacted with reagents, such as o-xylylene dibromide or similar compounds, the result of which is the formation of a stable covalent bridge through thioether bonds. The stable, thioether-bonded cyclic peptide may then be used as analytical tools, as reagents in ELISA assays, or as reagents for conjugation to an immunogenic carrier which may be comprised of polysaccharide, protein, both polysaccharide and protein, or any other material which confers enhanced immunogenicity on the cyclic peptide. Such conjugates are useful for inducing mammalian anti-peptide, anti-HIV, or HIV-neutralizing immune responses and for formulating vaccines to prevent HIV-Disease, including AIDS or ARC or for treating humans afflicted with AIDS or ARC.

## OBJECT OF THE INVENTION

Accordingly, it is an object of this invention to provide novel, stable, and cyclic HIV PND peptides which are useful as reagents for preparing conjugates which may be used to elicit mammlian anti-peptide, anti-HIV, or HIV-neutralizing immune responses, or to prepare compositions for use as anti-HIV vaccines or as immunogens for treatment of humans afflicted with AIDS. Another object is to provide a process for the stable cyclization of HIV PND peptides through thioether bonded structures.

## DEFINITIONS AND ABBREVIATIONS

| | |
|---|---|
| AA assay | amino acid analysis method wherein peptides or proteins are acid hydrolyzed to the free amino acids and then quantitated |
| Acm | acetamidomethyl thiol protecting group |
| activation | reaction of peptides, proteins, or polysaccharide moieties with a reagent capable of derivatizing the moiety in order to enable subsequent desirable reactions to occur |
| AIDS | Acquired Immune Deficiency Syndrome |
| amino acid | a molecule having both an acid and amino functional group; there are 20 common $\alpha$-amino acids with the general structure $H_2N-CHR-COOH$, wherein the R group defines the identity of the amino acid; these amino acids may have either a D or L stereochemical form and unless specified otherwise, by the lower-case one letter abbreviation, or the prefix D-before the amino acid name, the amino acid is of the natural or L configuration; the |

names of the 20 common amino acids and the structure of the R group are identified herein in single-letter code according to the following table:

| AMINO ACID NAME | 3-letter code | 1-letter code | side-chain (R) |
|---|---|---|---|
| Alanine | Ala | A | $-CH_3$ |
| Arginine | Arg | R | $-(CH_2)_3NHCHNH_2NH_2^+$ |
| Asparagine | Asn | N | $-CH_2CONH_2$ |
| Aspartic Acid | Asp | D | $-CH_2COOH$ |
| Cysteine | Cys | C | $-CH_2SH$ |
| Glutamic Acid | Glu | E | $-(CH_2)_2COOH$ |
| Glutamine | Gln | Q | $-(CH_2)_2CONH_2$ |
| Glycine | Gly | G | -H |
| Histidine | His | H | $-CH_2$-imidazole |
| Isoleucine | Ile | I | $-CH(CH_3)CH_2CH_3$ |
| Leucine | Leu | L | $-CH_2CH(CH_3)_2$ |
| Lysine | Lys | K | $-(CH_2)_4NH_3^+$ |
| Methionine | Met | M | $-(CH_2)_2SCH_3$ |
| Phenylalanine | Phe | F | $-CH_2$-Phenyl |
| Proline | Pro | P | $-\alpha$, N-$(CH_2)_3$ |
| Serine | Ser | S | $-CH_2OH$ |
| Threonine | Thr | T | $-CH(OH)CH_3$ |
| Tryptophan | Trp | W | $-CH_2$-indole |
| Tyrosine | Tyr | Y | $-CH_2$-phenyl-OH |
| Valine | Val | V | $-CH(CH_3)_2$ |

| | |
|---|---|
| antibody | a protein produced by mamalian B cells that is capable of binding a particular antigen |
| ARC | AIDS-Related Complex |
| AZT | Azidothymidine, an anti-AIDS compound |
| bigeneric spacer | a molecular chain resulting from the reaction of separately derivatized partners; analytical degradation of the conjugate formed through the spacer allows release and quantitation of the spacer, providing a measure of the degree of covalent attachment |
| capping | the elimination of reactive sites on a conjugate by reaction with small molecules |
| Cbz | benzyloxycarbonyl |
| conjugate | a complex of discrete chemical entities covalently bound one to the other, wherein at least one entity is a desired antigen (e.g. an HIV PND) and another entity is a carrier |
| Core amino acids | those amino acids of an HIV PND which are essential for inducing HIV-neutralizing immune responses in a mammal |
| DPPA | diphenylphosphorylazide |
| ELISA | enzyme-linked immunosorbant assay |
| Fmoc | 9-fluorenylmethyloxycarbonyl |
| HIV | Human Immunodeficiency Virus, a member of the lentivirus group and the |

| | |
|---|---|
| | purported etiologic agent implicated in AIDS and related complexes; HIV is alternatively known as HTLV (Human T-cell Lymphocyto-trophic Virus), LAV (Lymphadenopathy Associated Virus), and ARV (AIDS Related Virus) |
| immunogen | a molecule useful as a stimulator of a mammalian immune response |
| immunologically equivalent peptides | cyclic or linear peptides having in common the function of eliciting HIV neutralizing immune responses in mammals, such as antibodies which are able to recognize the equivalent peptide epitopes |
| marker amino acid | an amino acid having a signal in the AA assay which is free of interference by signals generated by other peptide or protein amino acids |
| Mtr | 4-methoxy-2,3,6-trimethylphenyl sulfonyl |
| NEM | N-ethylmaleimide |
| OMPC | Outer Membrane Protein Complex of Neisseria meningitidis; used as an immunoenhancer and peptide carrier |
| peptide | a polymer of amino acids linked by amide (peptide) bonds |
| PEP | peptide |
| PND | Principal Neutralizing Determinant; the name attributed to peptidyl sequences capable of high-affinity binding to HIV neutralizing antibodies and capable of raising HIV-neutralizing antibodies in a mammalian recipient upon inoculation with an immunogen containing the PND |
| PnPs6B | Streptococcus pneumoniae 6B capsular polysaccharide |
| PRO | an immunogenic protein |
| protein | a large peptide |
| PRP | Polyribosyl-ribitol phosphate |
| PSA | anionic polysaccharide, usually having repeat phosphate units in the monomer unit of the polymer |
| resins | solid support matrices for solid phase peptide synthesis<br>Wang:<br>4-(hydroxymethyl)phenoxymethyl linkage to copolystyrene-1%divinylbenzene resin, which is used for batch Fmoc solid phase peptide synthesis, with final 95% TFA cleavage from the resin and concomitant deprotection of acid sensitive side chain protecting groups;<br>Sasrin:<br>4-(hydroxymethyl)-3-methoxyphenoxymethyl linkage to copolystyrene-1%divinylbenzene resin, which is used for batch Fmoc solid phase peptide synthesis, with final 1% TFA/$CH_2Cl_2$ cleavage from the resin, leaving intact acid labile side chain protecting groups;<br>Pepsyn KA:<br>4-(hydroxymethyl)phenoxymethyl linkage to polyamide resin adsorbed on to kieselguhr, which is used for continuous flow column Fmoc solid phase peptide synthesis. Peptides are cleaved from the resin as described above for Wang resin;<br>Pepsyn KH:<br>4-(hydroxymethyl)-3-methoxymethyl linkage to polyamide resin adsorbed on to kieselguhr, which is used for Fmoc solid phase peptide synthesis. Side chain protected peptides are cleaved from the resin as described above for the Sasrin resin |
| "scaffold" | immunogen having multiple peptide epitopes built upon a carrier molecule |
| SCMHC | S-carboxymethyl homocysteamine, an acid-stable bigeneric spacer released by degradation of covalent conjugate immunogens and quantifiable by AA assay |
| SCMC | S-carboxymethyl cysteamine, an acid-stable bigeneric spacer released by degradation of covalent conjugate immunogens and quantifiable by AA assay |
| Z | benzyloxycarbonyl |

## DETAILED DESCRIPTION OF THE INVENTION

This invention is concerned with novel cyclic HIV PND peptides having the structure:

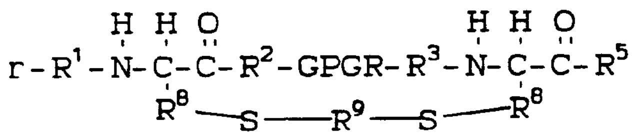

or pharmaceutically acceptable salts thereof, wherein:

r is:
a) hydrogen,
b) benzyloxycarbonyl,
c) Ac-Cys, or
d) Ac-Cys(Acm);

$R^1$ is:
a) a bond, or
b) a peptide of 1 to 5 amino acids, optionally including a marker amino acid;

$R^2$ is:
a) either a bond or a peptide of up to 17 amino acids if $R^3$ is a peptide of at least 2 amino acids, or
b) a peptide of between 2 to 17 amino acids, if $R^3$ is a bond;

$R^3$ is:
a) either a bond or a peptide of up to 17 amino acids if $R^2$ is a peptide of at least 2 amino acids, or
b) a peptide of between 2 to 17 amino acids, if $R^2$ is a bond;

-GPGR- is the tetramer -GlyProGlyArg-;

$R^5$ is:
a) -OH,
b) a peptide of 1 to 5 amino acids, optionally including a marker amino acid, or
c) $-NH_2$;

$R^8$ is lower alkyl of between one and eight carbons;

$R^9$ is:
a) $R^{10}$
b) xylylene; or

$R^{10}$ is:
a) lower alkyl of 1-8 carbons, or
b) $-CH_2-O-CH_2-$.

Lower alkyl consists of straight or branched chain alkyls having from one to eight carbons unless otherwise specified. Hereinafter, amino acids $-R^2$-GPGR-$R^3$-, which go toward formation of the loop of a cyclic peptide, will be referred to as loop amino acids. The term "nonlabile bond" means a covalent linkage, other than a labile disulfide bond, such as amide and thioether bonds. These covalent linkages may be to a bridge structure such as o-xylylene or a lower alkyl. By using an appropriate bridge structure, the conformation of the loop structure of the cycle may be optimized allowing the fine tuning of the PND epitope to be presented to the immune system. For example, use of an o-xylylene bridge generates a "tighter" loop structure than when an eight carbon straight chain alkyl is used as the bridge.

Accordingly, in one embodiment of the invention, the cyclic peptide having the structure:

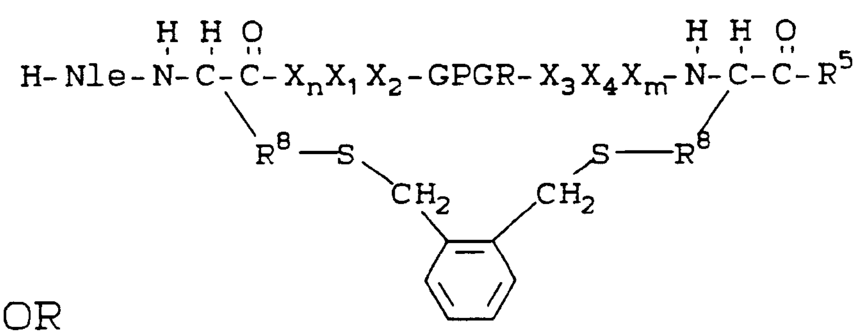

OR

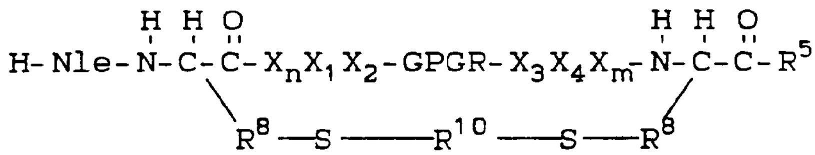

is prepared by cyclizing a linear peptide having the structure:

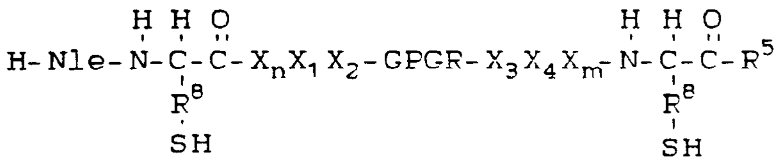

wherein:

-GPGR- is the tetramer -GlyProGlyArg-;

$X_1$ is a constituent of $R^2$ selected from:

a) serine,
b) proline,
c) arginine,
d) histidine,
e) glutamine, or
f) threonine;

$X_2$ is a constituent of $R^2$ selected from:

a) isoleucine,
b) arginine,
c) valine, or
d) methionine;

$X_n$ is a constituent of $R^2$ and is either a bond or a peptide of up to 15 amino acids;

$X_3$ is a constituent of $R^3$ selected from:

a) alanine,
b) arginine, or
c) valine;

$X_4$ is a constituent of $R^3$ and is selected from:

a) phenylalanine,
b) isoleucine,
c) valine, or
d) leucine;

$X_m$ is a constituent of $R^3$ and is a bond or a peptide of up to 15 amino acids; and

$R^{10}$ is a straight or branched chain lower alkyl of between one and eight carbons or $-CH_2OCH_2-$.

The novel cyclic peptides of this invention are prepared in essentially two phases: First the linear peptide is synthesized on a Milligen 9050 or an ABI-431A peptide synthesizer using 9-fluorenyl-methyloxycarbonyl (Fmoc) chemistry and appropriately side-chain protected Fmoc-amino acid pentafluorophenyl esters as reagents or using derivatized Wang resin, Fmoc chemistry, and side-chain protected Fmoc-amino acid symmetrical anhydrides, prepared in situ, as reagents.

Second, the linear peptide is cyclized, either in solution or with the peptide still attached to the solid phase resin by incorporating cysteine residues into the linear peptide at either end of the sequence which is to form the loop, and treating the peptide with o-xylylene dibromide or similar reagent, such as the diiodide, dichloride, or a dihalogenated lower alkyl having from one to eight carbon atoms, which reacts with the sulfur atoms of the cysteines to form a cyclic structure containing two nonlabile thioether bonds.

Products obtained may be characterized by fast atom bombardment-mass spectrometry [FAB-MS], reverse phase HPLC, amino acid analysis or nuclear magnetic resonance spectroscopy (NMR).

Thus, the peptides of this invention may be prepared as further described below in (i) and (ii):

i. Peptide Cyclization in the Solid State:

A linear peptide containing $C^1$ and $C^2$ on either side of the loop amino acids, where $C^1$ and $C^2$ are both cysteine or another amino acid containing free sulfhydryl groups in the side chain, is prepared according to known synthetic procedures (see discussion supra). In the completed cyclic PND, the sulfhydryl containing side chains, ($-R^8$-SH), go toward making up the $-R^8$-S-groups of the completed cyclic HIV PND structure shown above. Amino acids to be incorporated which have reactive side chains (R groups) are used in an appropriately R-group protected form. For example, histidine is triphenylmethyl (Trt) protected, and arginine is 4-methoxy-2,3,6-trimethylphenyl sulfonyl (Mtr) protected.

Preferably, a resin is purchased with $C^2$ in its Acm protected form already attached to the resin, for example, Fmoc-L-Cys(Acm)-O-Wang resin. The cysteine incorporated at the amino terminal side of the loop amino acids, $C^1$, may also be the Acm derivative. Either $C^1$ or $C^2$ may be bound to additional amino acids, $R^1$ or $R^5$ respectively, which may be utilized in the formation of conjugates with carrier molecules or may serve as marker amino acids for subsequent amino acid analysis, such as when norleucine or ornithine is used.

The sulfur of the acetamidomethylated cysteines are reacted, at room temperature for about 15 hours in a solvent compatible with the resin, as a 1-50% concentration of an organic acid, preferably about 10% acetic acid in anhydrous dimethylformamide (DMF), with about a four fold molar excess of a heavy metal salt, such as mercuric acetate [$Hg(OAc)_2$] for each Acm group. The resulting heavy metal thioether, for example the mercuric acetate thioether of the peptide, PEP(S-HgOAc), is then washed and dried. Addition of excess hydrogen sulfide in DMF yields insoluble metal sulfide, e.g. mercuric sulfide (HgS), and the peptide with free sulfhydryl groups.

A mixture of about an equimolar amount, as compared with peptide, of o-xylylene dibromide or dichloride, or a dibrominated or dichlorinated lower alkyl, or similar reagent which will provide a desirable bridge length, is added to the derivatized resin. A large excess of a tertiary amine, preferably triethylamine ($NEt_3$) in DMF, is then added slowly. The reaction with the bis-sulfhydryl peptide moiety is allowed to proceed for about sixteen hours at room temperature, yielding the bridge group derivatized cyclic peptide bound to resin. Deprotection of the Fmoc amino terminus is accomplished over about 20 minutes by addition of a base, such as 20% piperidine in DMF, followed by washing and drying. Deprotection of acid sensitive side chain protecting groups and cleavage from the resin is achieved by treatment with 95% trifluoroacetic acid (TFA) in the presence of 4% 1,2-ethanedithiol and 1% thioanisole. The dissolved cyclic peptide may then be separated from the resin by filtration. The filtrate is evaporated and the crude residual product is purified by high performance liquid chromatography (HPLC) according to known methods, for example by reverse phase HPLC.

ii. Cyclization of Peptides in Solution:

Essentially the same process described above for solid state cyclization applies with two main variants: If the peptide is cleaved (95% TFA/4% ethanedithiol/1% thioanisole) from a pepsyn KA resin, acid labile side chain protecting groups are also removed, including Cys(Trt) which provides the necessary tree -SH function. If however, Cys(Acm) protection is used, then mercuric acetate/hydrogen sulfide cleavage to the free -SH group is required as an independent procedure, with the linear peptide either on or off the resin.

The preferred method however, is the use of Cys(Acm) protection and Sasrin or Pepsyn KH resin, and cleavage of the linear, fully protected peptide from the resin with 1% $TFA/CH_2Cl_2$. Mercuric acetate/ hydrogen sulphide then selectively converts Cys(Acm) to the free -SH group, and cyclization is effected on the otherwise protected peptide. Acid labile side chain protecting groups are cleaved with 95% TFA/4% ethanedithiol/1% thioanisole, and the cyclic peptide is isolated by HPLC.

Removal of excess reagents, such as unreacted xylylene dibromide, prior to acid cleavage of the protecting groups is conveniently achieved by, for example, a step gradient reverse phase HPLC run prior to more selective gradient elution.

The following examples are provided to further define the cyclic peptide invention and to more particularly demonstrate how to make and use such peptides. However, the examples provided are not to be construed so as to limit the scope of the invention.

EXAMPLE 1

Solution Cyclization to Form cPND1:

Anhydrous DMF (20 ml) was degassed and HPLC-isolated linear 11-mer H-NleCHIGPGRAFC-OH (20 mg, 17 $\mu$mole) was dissolved and sealed under a nitrogen atmosphere. A solution of o-xylylene dibromide (4.7 mg, 17.9 $\mu$mole) in anhydrous DMF (50 $\mu$l) was added. Next, $NEt_3$ (11.9 $\mu$l, 85.2 $\mu$mole) in DMF was added over a

period of about 6.5 hours. About one hour after complete addition of the base, the solution was dried. The residue was resuspended in ether, centrifuged to spin down the insoluble product, and then redried. An aliquot analyzed by fast atom bombardment-mass spectrometry (FAB-MS) yielded a major ion $[M+H]^+$ of 1275. Isocratic reversed-phase HPLC on a Vydac Cl8 column (0.46 x 25.0 cm) using 0.1% TFA/24% $CH_3CN$ at 2.0 ml-/minute, monitored at 215 nm, showed a sharp product peak having a retention time of about 18.5 minutes. A preparative scale isolation was run over 135 minutes at 10 ml/minute from 24-29% $CH_3CN$, and the product with a retention time of 76.41 minutes under these conditions was collected and rechromatographed under analytical conditions to confirm its identity. Amino acid analysis and 400 MHz NMR analyses were consistent with the structure cPND1:

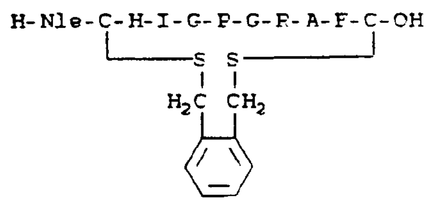

EXAMPLE 2

Solution Cyclization to Form cPND2:

The procedure described in Example 1 was followed except that the linear 10-mer H-Nle-CIGPGRAFC-OH (20 mg, 19.3 μmole) was used. After the addition of base, the solution was kept under nitrogen for an additional 9 hours. FAB-MS yielded $[M+H]^+$ = 1138 and $[M+Na]^+$ = 1160, consistent with the structure proposed for cPND2. Preparative HPLC using two 2.12 x 25 cm Vydac Cl8 columns was conducted over 90 minutes from 24% to 28 % CH3CN/0.1% TFA, at 10 ml/minute. The eluate was monitored at 215 nm and two product peaks at retention times of 63.42′ and 70.82′ were collected, dried, and sujected to FAB-MS. Both materials had $[M+H]^+$ of 1138, (the later peak also had a $[M+Na]^+$ of 1160) which is consistent with the structure cPND2:

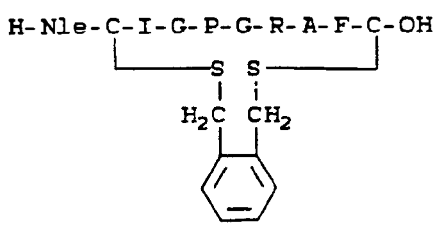

EXAMPLE 3

Solution Cyclization to Form cPND3:

The same procedure used in Examples 1 and 2 was employed here except that the linear 15-mer, H-Nle-CRIQRGPGRAFVTC-OH (21.2 mg, 11.92 μmole) was used, and $NEt_3$ was added over about 10 hours. An additional 5-hour exposure to base was permitted prior to analysis by analytical HPLC. FAB-MS of the crude product showed a single intense $[M+H]^+$ of 1779 which is consistent with the structure of cPND3:

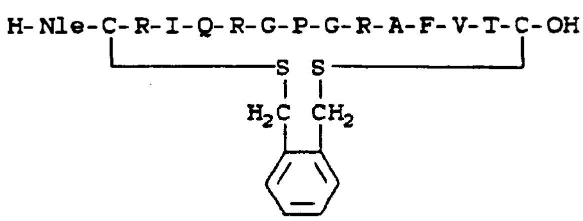

EXAMPLE 4

Solid State Cyclization to Form cPND1:

A linear PND peptide was prepared on Wang resin on an ABI-431A peptide synthesizer, starting from Fmoc-L-Cys(Acm)-O-Wang resin (0.61 meq/gram). Fmoc chemistry and Fmoc-amino acid symmetrical anhydrides (4x excess, prepared in situ) were used as reagents on a 0.25 mmole scale to generate 745 mg of the peptide:

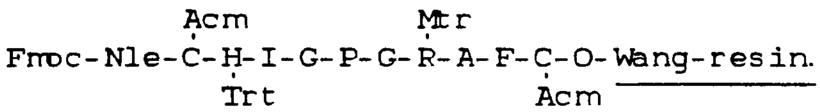

$Hg(OAC)_2$ (64 mg, 0.2 mmole) was dissolved in 10% acetic acid in DMF (0.5 ml) and added to the dried resin (149 mg, 0.05 meq) shown above. More 10% acetic acid in DMF (0.2 ml) was added to the swollen resin, and the solution was stirred overnight. Thereafter the resin was filtered, washed with DMF (5 x 1 ml), $CH_2Cl_2$ (3 x 1 ml), and ether (3 x 2 ml). Subsequently, the resin was dried and 1 ml $H_2S$ saturated DMF was added, followed by a second aliquot of the same. The resin was then filtered and washed as above and then dried, yielding a black resinous powder (179 mg).

A mixture of o-xylylene dibromide (3.2 mg) and $NEt_3$ (3.4 µl) in DMF (2 ml) was added to the resin (35 mg) at room temperature, and allowed to react for 16 hours. The resin was filtered, washed as above, and dried. The Fmoc was removed by treatment with 20% piperidine in DMF (1 ml) over 20 minutes at room temperature. The resin was washed again as above, and dried.

The cPND1 peptide was cleaved from the resin, and Trt and Mtr protecting groups concomitantly removed, by treating with 95% TFA/4% ethane dithiol/1% thioanisole (1 ml) at room temperature over 6 hours. The solution was filtered, the resin washed with additional 100% TFA (3 x 1 ml), and the combined filtrate was evaporated at 20°C/0.1 mm pressure. Material that was soluble in ether was removed by extraction (3 x 2 ml) and the insoluble crude product was redried at 20°C/0.1mm pressure.

Analytical HPLC using a 0.46 x 25 cm Vydac Cl8 column was used to identify the product. Comparison with the product obtained from Example 1 confirmed that authentic product was present (retention time of 12.88' as compared with 12.97'). Preparative HPLC was conducted over 90 minutes from 25% to 30% $CH_3CN$/0.1% TFA at 10 ml/minute using two 2.12 x 25 cm Vydac Cl8 columns in series. The peak eluting at 54.12' was collected. Co-chromatography, on an analytical scale, of this material with material prepared in Example 1 showed a single sharp peak. FAB-MS had a $[M+H]^+$ of 1275, confirming the preparation of cPND1 (see Example 1 above for structure).

EXAMPLE 5

Preparation of HIV PND Peptides Through Lower Alkyl Bridges:

A linear peptide, such as any of those used in Examples 1-4, is cyclized by addition of a dibrominated lower alkyl, having between one and eight carbons, straight or branched chain. For example methylene dibromide, 1,2-dibromoethane, or 1,3-dibromopropane, is added to a linear HIV PND peptide having two free sulfhydryls groups. The procedure of Mosberg, H.I., and Omnaas, J. R., J. Am. Chem. Soc., 107, 2986 (1985) may be used to generate the novel cyclic HIV PND peptides of this embodiment of the invention. Examples of preferred compounds of this embodiment are:

| Name | Structure |
|---|---|
| cPND33 | 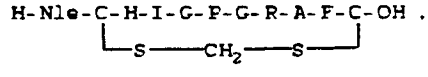 |
| cPND34 | 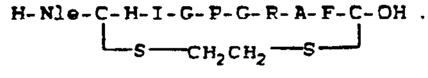 |

EXAMPLE 6

Preparation of HIV PND peptides through Oxy-dimethylene Bridges:

The procedure of Omnass and Mosberg may be modified by replacing lower alkyl dibromide with bis-(chloromethyl)ether, thereby forming an oxydimethylene bridge between the two sulfur atoms of the two cysteines. An example of a compound which may be prepared with the $-CH_2-O-CH_2-$ bridge structure is:

| Name | Structure |
| --- | --- |
| cPND35 | 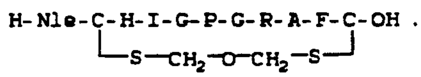 |

While the foregoing specification teaches the principles of the present invention, with examples provided for the purpose of illustration, it will be understood that the practice of the invention encompasses all the usual variations, adaptations, modifications, or deletions as come within the scope of the following claims and its equivalents.

## Claims

**1.** A cyclic HIV PND peptide having the structure:

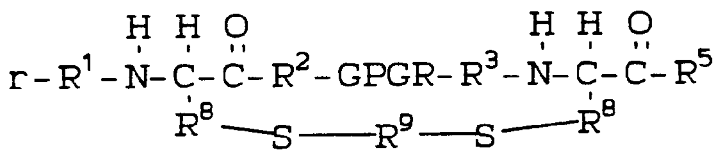

or pharmaceutically acceptable salts thereof, wherein:

r is:

a) hydrogen,
b) benzyloxycarbonyl,
c) Cys(Ac), or
d) AcmCys(Ac);

$R^1$ is:

a) a bond, or
b) a peptide of 1 to 5 amino acids, optionallly including a marker amino acid;

$R^2$ is :

a) either a bond or a peptide of up to 17 amino acids if $R^3$ is a peptide of at least 2 amino acids, or
b) a peptide of between 2 to 17 amino acids, if $R^3$ is a bond;

$R^3$ is:

a) either a bond or a peptide of up to 17 amino acids if $R^2$ is a peptide of at least 2 amino acids, or
b) a peptide of between 2 to 17 amino acids, if $R^2$ is a bond;

-GPGR- is the tetramer -GlyProGlyArg-;

$R^5$ is:

a) -OH,
b) a peptide of 1 to 5 amino acids, optionally including a marker amino acid, or
c) $-NH_2$;

$R^8$ is a lower alkyl of between one and eight carbons;

$R^9$ is:

a) $R^{10}$, or
b) xylylene; and

R10 is lower alkyl or $-CH_2OCH_2-$.

**2.** The peptide of Claim 1 having the structure:

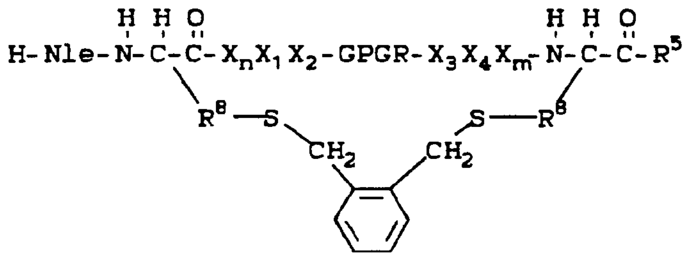

or pharmaceutically acceptable salts thereof, wherein:

-GPGR- is the tetramer -GlyProGlyArg-;

$X_1$ is a constituent of $R^2$ selected from:

a) serine,
b) proline,
c) arginine,
d) histidine,
e) glutamine, or
f) threonine;

$X_2$ is a constituent of $R^2$ selected from:

a) isoleucine,
b) arginine,
c) valine, or
d) methionine;

$X_n$ is is a constituent of $R^2$ and is either a bond or a peptide of up to 15 amino acids;

$X_3$ is a constituent of $R^3$ selected from:

a) alanine,
b) arginine, or
c) valine;

$X_4$ is a constituent of $R^3$ and is selected from;

a) phenylalanine,
b) isoleucine,
c) valine, or
d) leucine; and

$X_m$ is a constituent of $R^3$ and is a bond or a peptide of up to 15 amino acids.

3. The peptide of Claim 1 having the structure:

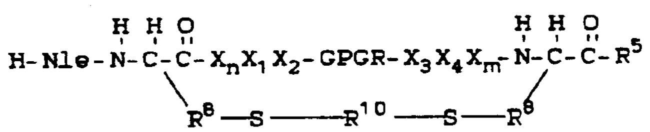

or pharmaceutically acceptable salts thereof, wherein:

-GPGR- is the tetramer -GlyProGlyArg-;

$X_1$ is a constituent of $R^2$ selected from:

a) serine,
b) proline,
c) arginine,
d) histidine,
e) glutamine, or
f) threonine;

$X_2$ is a constituent of $R^2$ selected from:

a) isoleucine,
b) arginine,
c) valine, or
d) methionine;

$X_n$ is is a constituent of $R^2$ and is either a bond or a peptide of up to 15 amino acids;

$X_3$ is a constituent of $R^3$ selected from:
a) alanine,
b) arginine, or
c) valine;
$X_4$ is a constituent of $R^3$ and is selected from:
a) phenylalanine,
b) isoleucine,
c) valine, or
d) leucine;
$X_m$ is a constituent of $R^3$ and is a bond or a peptide of up to 15 amino acids; and
$R^{10}$ is a straight or branched chain lower alkyl of between one and eight carbons or $-CH_2OCH_2-$.

4. The peptide of Claim 2 having the structure:

| Name | Structure |
|---|---|
| a) cPND1 | 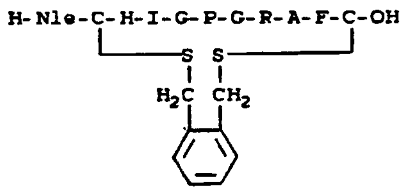 , |
| b) cPND2 | 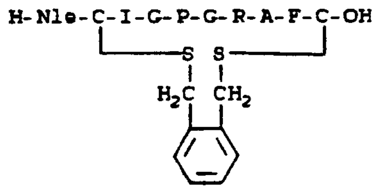 , |
| c) cPND3 | 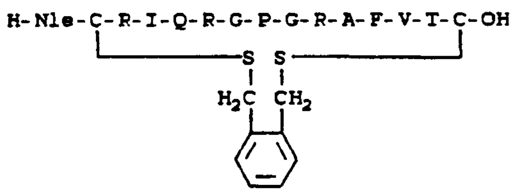 , |
| d) cPND33 | 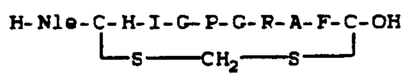 , |
| e) cPND34 | 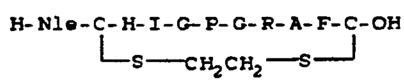 , or |
| f) cPND35 | 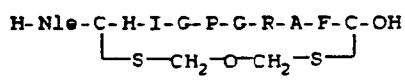 . |

5. A process for makinp the peptide of Claim 1 which comprises the steps of (a) synthesizing a linear HIV PND peptide containing side-chain protected amino acids and Acm derivatized cysteines on either side of the loop amino acids $-R^2$-GPGR-$R^3-$; (b) reacting the Acm derivatized cysteines with a heavy metal to form the heavy metal-sulfo-cysteine derivative; (c) reacting the product of step (b) with $H_2S$ to liberate the free thiol groups; and (d) reacting the dithiol product of step (c) with a bifunctional bridge group selected from among: lower alkyl dihalide, o-, m-, or p-xylylene dihalide, where the alkyl is a lower alkyl, and the halogen is bromine, chlorine, or iodine.

6. The process of Claim 5 for making a peptide having the structure:

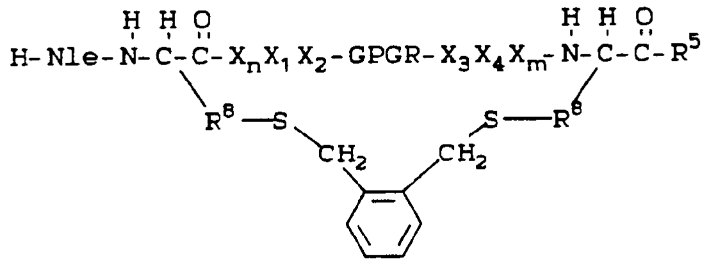

or pharmaceutically acceptable salts thereof, wherein:

-GPGR- is the tetramer -GlyProGlyArg-;

$X_1$ is a constituent of $R^2$ selected from:

a) serine,
b) proline,
c) arginine,
d) histidine,
e) glutamine, or
f) threonine;

$X_2$ is a constituent of $R^2$ selected from:

a) isoleucine,
b) arginine,
c) valine, or
d) methionine;

$X_n$ is is a constituent of $R^2$ and is either a bond or a peptide of up to 15 amino acids;

$X_3$ is a constituent of $R^3$ selected from:

a) alanine,
b) arginine, or
c) valine;

$X_4$ is a constituent of $R^3$ and is selected from:

a) phenylalanine,
b) isoleucine,
c) valine, or
d) leucine; and

$X_m$ is a constituent of $R^3$ and is a bond or a peptide of up to 15 amino acids;

which consists essentially of the steps of (a) preparing a linear, Acm-thiol protected peptide having the structure

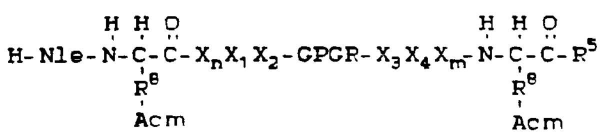 ;

(b) reacting the peptide of step (a) with mercuric acetate; (c) reacting the product of step (b) with $H_2S$; and (d) contacting the product of step (c) with xylylene dibromide, dichloride, or diiodide.

**7.** The process of Claim 5 for making a peptide having the structure:

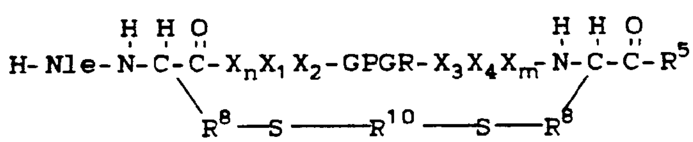

or pharmaceutically acceptable salts thereof, wherein:

-GPGR- is the tetramer -GlyProGlyArg-;

$X_1$ is a constituent of $R^2$ selected from:

a) serine,
b) proline,
c) arginine,
d) histidine,
e) glutamine, or
f) threonine;
$X_2$ is a constituent of $R^2$ selected from:
a) isoleucine,
b) arginine,
c) valine, or
d) methionine;
$X_n$ is is a constituent of $R^2$ and is either a bond or a peptide of up to 15 amino acids;
$X_3$ is a constituent of $R^3$ selected from:
a) alanine,
b) arginine, or
c) valine;
$X_4$ is a constituent of $R^3$ and is selected from:
a) phenylalanine,
b) isoleucine,
c) valine, or
d) leucine;
$X_m$ is a constituent of $R^3$ and is a bond or a peptide of up to 15 amino acids; and
$R^{10}$ is a straight or branched chain lower alkyl of between one and eight carbons; or $-CH_2OCH_2-$
which consists essentially of the steps of (a) preparing a linear, Acm-thiol protected peptide having the structure

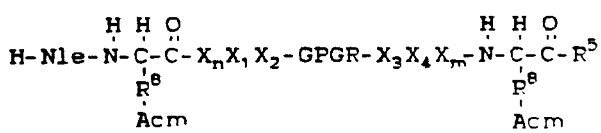

:

(b) reacting the peptide of step (a) with mercuric acetate; (c) reacting the product of step (b) with $H_2S$; and (d) contacting the product of step (c) with a straight or branched chain lower alkyl dibromide, dichloride, or diiodide; or with 1,3-dihalomethylether.

**8.** The process of Claim 6 for making a peptide having the structure:

| Name | Structure |
|---|---|
| a) cPND1 | 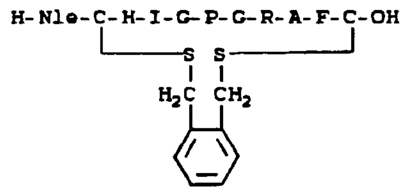 , |
| b) cPND2 | 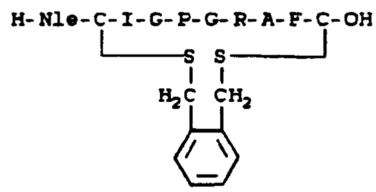 , |
| c) cPND3 | 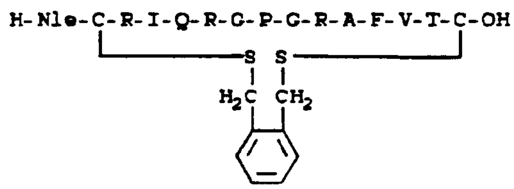 , |
| d) cPND33 | 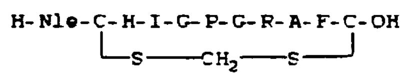 , |
| e) cPND34 | 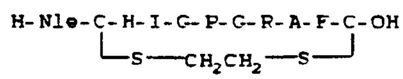 , or |
| f) cPND35 | 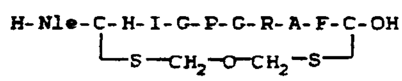 . |